# EUROPÄISCHE PATENTSCHRIFT

(11) **EP 0 399 163 B1**
(45) Veröffentlichungstag und Bekanntmachung des Hinweises auf die Patenterteilung: **21.04.1993**
(21) Anmeldenummer: 90105492.4
(22) Anmeldetag: 23.03.1990
(51) Int. Cl.: B22C 7/02, B22C 9/00

(54) **Verfahren zur Herstellung offenzelliger metallischer Strukturen**
Process for fabrication of open-cell metallic structures
Procédé pour fabriquer des structures métalliques à alvéoles ouvertes

(30) Priorität: 26.05.1989 DE 3917033
(43) Veröffentlichungstag der Anmeldung: 28.11.1990
(73) Patentinhaber: ESKA MEDICAL LÜBECK MEDIZINTECHNIK GmbH & Co., D-23556 Lübeck (DE)
(72) Erfinder: Ahlers, Olaf, D-2000 Hamburg 79 (DE)
(74) Vertreter: Weiss, Christian, Dipl.-Ing.

(56) Entgegenhaltungen:
- AT-B- 341 690
- DE-A- 3 106 917
- DE-A- 3 502 504
- DE-C- 2 715 204
- DE-C- 3 224 265
- US-A- 3 747 663

## Beschreibung

Die Erfindung betrifft ein Verfahren zur Herstellung offenzelliger metallischer Strukturen unter Anwendung eines verlorenen Positivmodells aus einem offenporigen Kunststoffträger mit einer Durchschnittsweite der Poren von 1 bis 3 mm, bei dem nach Herstellung eines Negativmodells dessen Kernräume durch Gießen mit Metall gefüllt werden und schließlich das Negativmodell erfernt wird.

Ein Verfahren dieser Art, allerdings zur Herstellung eines Implantates als Knochenersatz, ist aus der DE-OS 31 06 917 bekannt. Hierbei werden die Hohlräume eines das Positivmodell bildenden Natur- oder Kunststoffschwammes mit einer keramischen Einbettmasse gefüllt, das Modellmaterial unter Anwendung von Hitze entfernt und die Hohlräume des so gewonnenen Negativmodells mit Metall gefüllt. Schließlich wird das keramische Kernmaterial entfernt.

Als problematisch bei diesem Verfahren hat sich ergeben, daß die Wandungen und die vernetzten Stege eines Natur- oder Kunststoffschwammes zu schwach sind, so daß der mittels des Verfahrens hergestellte metallische Formkörper eine nur unbefriedigende Stabilität aufweist.

Gemäß der DE-OS 32 24 265 ist das vorerwähnte Verfahren dadurch weitergebildet worden, daß die Wandungen und/oder vernetzten Stege der Poren des hierbei als Positivmodell verwandten Kunststofformkörpers bei Raumtemperatur entweder durch Eintauchen des Modells in verflüssigtes Wachs oder in eine Wachs-Wasser-Emulsion oder durch Einsprühen des Modells mit verflüssigtem Wachs oder einer Wachs-Wasser-Emulsion in der Dicke durch sich niederschlagendes Wachs verstärkt werden, worauf das Wasser verdampft und anschließend das verbleibende trockene Wachs durch eine Kunststofflackbeschichtung geschützt wird.

Dieses Verfahren gestattet es in gewissen Grenzen, einen offenzelligen metallischen Formkörper herzustellen, bei dem die Stege der Zellen dicker sind als bei dem mit erstgenannten Verfahren hergestellten Formkörper.

Als nachteilig hat sich bei diesem Verfahren erwiesen, daß sich das mittels Eintauchen oder durch Einsprühen auf bzw. in den Kunststofformkörper gebrachte Wachs in Verbindung mit dem aufgesprühten Lack gegenüber dem elastischen Kunststofformkörper starr verhält, so daß sich das Wachs unter Druck wieder leicht von den Wandungen und Stegen der Poren des Formkörpers lösen kann. Ein weiterer wesentlicher Nachteil dieses Auftragverfahrens ist darin zu sehen, daß sich die Polymeroberflächen der Wandungen und Stege des Kunststofformkörpers negativ gegenüber dem Wachs oder der Wachs-Wasser-Emulsion aufladen. Infolgedessen kommt es zu keinem zirkulär-homogenen Verbund zwischen den Wandungen und Stegen des Kunststofformkörpers und dem Wachs. Ins besondere trifft dies zu auf die Wandungen und Stege, die sich in der räumlichen Tiefe des Formkörpers, also von dessen Oberfläche entfernt befinden. Das Fließverhalten des Wachses oder der Wachs-Wasser-Emulsion in dem Formkörper ist also nicht so günstig, als daß auch die Wandungen und Stege in der räumlichen Tiefe des Positivmodells in befriedigendem Maße verstärkt werden. In der Folge weist dementsprechend der mit dem Verfahren hergestellte metallische Formkörper in seiner räumlichen Tiefe Wandungen und Stege auf, die teilweise zu dünn sind, um auftretenden Belastungen dauerhaft standhalten zu können.

Vor dem aufgezeigten Hintergrund ist es nun die Aufgabe der vorliegenden Erfindung, das eingangs erwähnte Verfahren so weiterzubilden, daß die Wandungen und Poren des Positivmodells durchgängig, das heißt also auch in dessen räumlicher Tiefe zuverlässig verstärkt werden.

Gelöst wird diese Aufgabe durch die Verfahrensschritte gemäß dem kennzeichnenden Teil des Anspruches 1.

Demgemäß wird nach erfolgter Reinigung des als Positivmodell dienenden Kunststoffträgers dieser durchgängig mit einem seine Oberflächenstrukturen anlösenden haftungsvermittelnden Harzfilm benetzt, wonach mindestens eine Schicht eines selbstvernetzenden Zweikomponenten-Silikons durchgängig in bzw. auf den Kunststoffträger ein- bzw. aufgetragen wird zur Verstärkung der Wandungen und/oder vernetzten Stege des porigen Kunststoffträgers.

Die Reinigung des Kunststoffträgers kann beispielsweise mittels Aceton vorgenommen werden.

Der haftungsvermittelnde Harzfilm wird beispielsweise mit einer Spritzpistole ein- bzw. aufgetragen. Der Harz wird in einem Lösungsmittel gelöst sein, nach dessen Verdampfen der haftungsvermittelnde Harzfilm auf den Oberflächenstrukturen des Kunststoffträgers zurückbleibt. Hernach wird das Zweikomponenten-Silikon beispielsweise mit einer Spritzpistole oder durch Betupfen in- bzw. auf den Kunststoffträger ein- bzw. aufgetragen.

Die Verwendung des haftungsvermittelnden Harzfilmes ermöglicht einen zirkulären Vernetzungsverbund zwischen den Stegen und Wandungen des Kunststoffträgers und dem Zweikomponenten-Silikon.

Nach dem Vernetzen des Zweikomponenten-Silikons schließen sich die bekannten Verfahrensschritte zur Herstellung eines Negativmodelles an. Namentlich werden die Poren des wie zuvor beschrieben behandelten Kunststoffträgers mit einer keramischen Einbettmasse gefüllt, die zu einem Kern unter Verflüchtigung des Positivmodelles gebrannt wird. Dessen Kernräume werden durch Gießen mit Metall gefüllt, wonach das Kernmaterial, also das Negativmodell, entfernt wird.

Es wird betont, daß die mit dem erfindungsgemäßen Verfahren hergestellten offenzelligen metallischen Strukturen vielfältig einsetzbar sind. So können sie beispielsweise Teile eines Körperimplantates bilden. Gegenüber einem nach dem Verfahren der schon genannten DE-OS 32 24 265 hergestellten Implantat als Knochenersatz weist die mit vorliegendem Verfahren hergestellte metallische Struktur größere Poren und an ihrer Außenseite schärfere Kanten auf. Die größeren Poren ermöglichen aufgrund ihrer Dimensionierung das Einwachsen von Knochenbälkchen auch in ihre räumliche Tiefe hinein. Die Scharfkantigkeit ergibt sich daraus, daß die Wahrscheinlichkeit bei Zuschnitt des Positivmodells eines Durchtrennens der größeren Poren wesentlich größer ist als beim Zuschnitt des Positivmodells aus einem Kunststoffträger mit kleineren Poren. Die Scharfkantigkeit der Außenfläche einer offenzelligen Struktur bei Knochenimplantaten regt das Knochenwachstum in die offenzellige Struktur an.

Daneben ist der Einsatz der nach dem erfindungsgemäßen Verfahren gewonnenen Strukturen auch beispielsweise als Filter in Form einer künstlichen Niere möglich und vorteilhaft.

Das erfindungsgemäße Verfahren kann vorteilhaft weitergebildet werden durch die Ergänzung durch einen weiteren Verfahrensschritt nach der Vernetzung des Zweikomponenten-Silikons. Zur zusätzlichen Versteifung der Oberflächenstruktur des im Kunststoffträger befindlichen Silikon-Netzwerkes kann ein Zweikomponenten-Polyurethanharz auf bzw. eingetragen werden. Zur Herstellung eines innigen Verbundes zwischen dem Zweikomponenten-Silikon und dem aufzutragenden Zweikomponenten-Polyurethanharz hat das letztere aufgrund seiner Zusammensetzung eine die Oberfläche des Zweikomponenten-Silikons leicht anlösende Eigenschaft.

Der haftungsvermittelnde Harzfilm besteht vorzugsweise aus einem ungesättigten Silikonharz. Hierdurch ergibt sich ein System, bei dem ein ungesättigtes Silikonharz dem ein-bzw. aufzutragenden Zweikomponenten-Silikon gegenübersteht, dessen Gemisch regelmäßig ebenfalls ungesättigt sein wird.

Hierdurch wird eine regelrechte Saugwirkung (Anziehung) zwischen dem Silikonharz und dem Zweikomponenten-Silikon erzielt, wodurch in besonders eindrucksvoller Weise die Stege und Wandungen der Poren des Kunststoffträgers auch in dessen räumlicher Tiefe verstärkt werden.

Das Zweikomponenten-Silikon ist vorteilhafterweise ein raumtemperaturvernetzender, ungesättigter Zweikomponenten-Silikonkautschuk. Grundsätzlich kann die Vernetzung natürlich auch durch Abschrecken des mit dem Zweikomponenten-Silikon versehenen Kunststoffträgers in Wasser erzielt bzw. beschleunigt werden.

Oftmals wird die Notwendigkeit bestehen, die Wandungen und/oder Stege der Poren des Kunststoffträgers durch Auf- bzw . Eintragen mehrerer Schichten des Zweikomponenten-Silikons zu verstärken. Hierbei sind die einzelnen Schichten vorteilhaft verschiedenfarbig. Dies ermöglicht es, die Schichtdicken beispielsweise unter einer Lupe erkennbar zu machen, um die Gleichmäßigkeit der Schichtdicken kontrollieren zu können. Diese Kontrolle dient der Sicherung der Qualität der mit dem erfindungsgemäßen Verfahren hergestellten offenzelligen metallischen Strukturen. Eine gleichmäßige Verstärkung der Wandungen und/oder vernetzten Stege der Poren des Kunststoffträgers spiegelt sich nach Durchführung des Verfahrens wider in der Gleichmäßigkeit der Wandungen und/oder Stege der metallischen Struktur.

Der Vorteil des erfindungsgemäßen Verfahrens gegenüber dem Verfahren gemäß dem Stand der Technik manifestiert sich in dem mit ihm hergestellten Produkt. Aus diesem Grunde wird der Vorteil anhand der schematischen Darstellungen von offenzelligen metallischen Strukturen erläutert werden. Hierbei zeigt:
- Figur 1a:: eine schematische Darstellung einer Schnittansicht durch einen offenzelligen Belag, hergestellt durch die bekannten Verfahren;
- Figur 1b:: eine Aufsicht auf den Belag gemäß Figur 1a;
- Figur 2a:: eine schematische Darstellung einer Schnittansicht durch einen offenzelligen Belag, hergestellt nach dem vorliegenden Verfahren;
- Figur 2b:: eine Aufsicht auf den Belag gemäß Figur 2a.

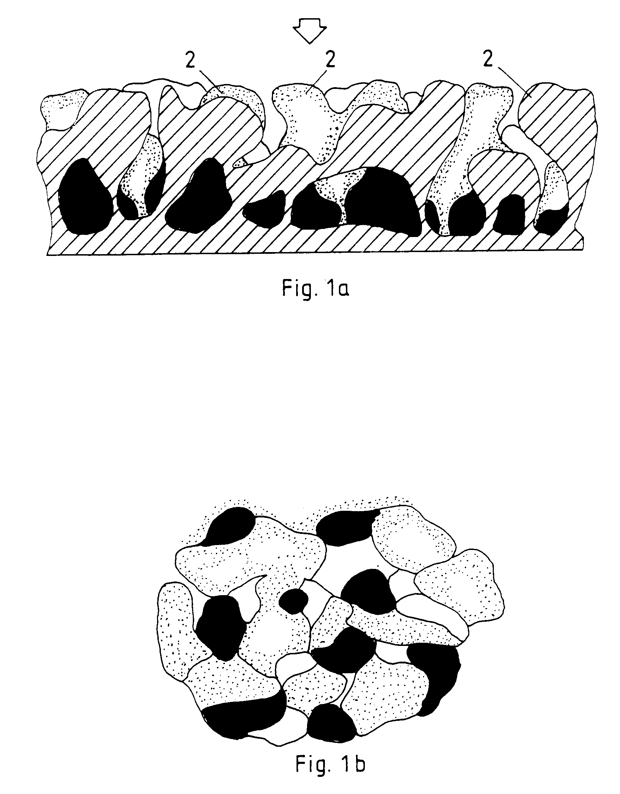

Deutlich erkennbar in Figur 1a ist, daß die Wandungen und vernetzten Stege der Poren mit zunehmender räumlicher Tiefe (in Richtung des eingetragenen Pfeiles) abnimmt. Dies ist - wie bereits oben erwähnt - eine Folge des schlechteren Fließverhaltens des flüssigen Wachses oder der Wachs-Wasser-Emulsion in dem als Positivmodell verwandten Kunststoffformkörper.

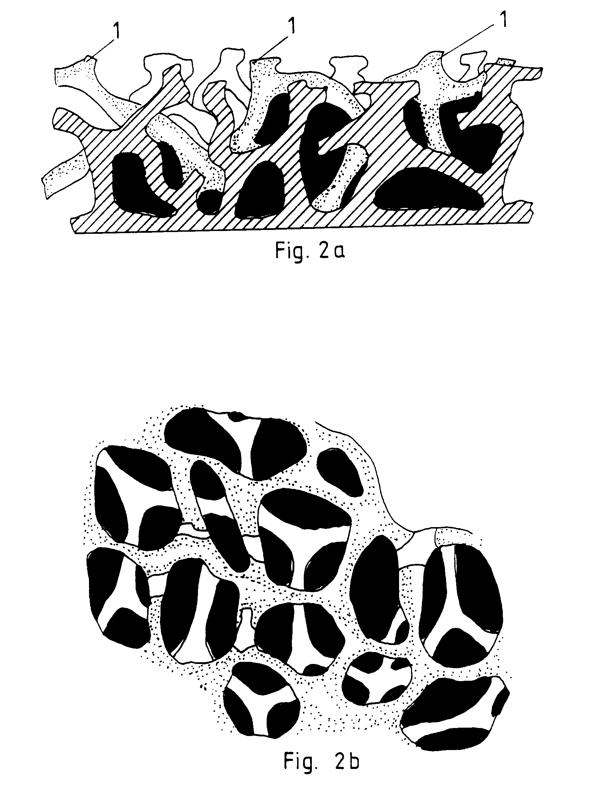

Demgegenüber ist in Figur 2a deutlich erkennbar, daß die Wandungen und Stege in der Struktur, hergestellt nach dem vorliegenden Verfahren, auch in der räumlichen Tiefe eine ausreichende Stärke aufweisen. Dies ist - wie oben ausgeführt - eine Folge des Auftragens des haftungsvermittelnden Harzfilmes auf bzw. in den als Positivmodell verwendeten Kunststoffträger in Verbindung mit der Verstärkung der Wandungen und vernetzten Stege des Kunststoffträgers durch das Zweikomponenten-Silikon.

Figur 1b zeigt die Aufsicht auf eine nach dem bekannten Verfahren hergestellte Struktur. Deutlich erkennbar ist, daß an der Oberfläche sehr viel Material zurückgeblieben ist, welches an anderer Stelle, nämlich in der räumlichen Tiefe, fehlt. Darüber hinaus sind die Poren in der Struktur relativ klein. Dies schwert unter Umständen das Einwachsen von Knochenbälkchen in die Struktur, welche Teil eines Knochenimplantates ist.

Demgegenüber ist aus Figur 2b deutlich erkennbar, daß das Material nicht zum größten Teil an der Oberfläche der Struktur angereichert ist. Darüber hinaus sind die Poren oder Zellen der Struktur relativ groß. Wird die Struktur beispielsweise angewendet bei Knochenimplantaten, so ist das Einwachsen von Knochenbälkchen ohne weiteres möglich. Hingewiesen sei schließlich auf eine gewisse Scharfkantigkeit der Oberfläche der nach dem vorliegenden Verfahren hergestellten Struktur, die sich aus dem Vorhandensein von Zacken 1 ergibt. Demgegenüber ist die Oberfläche der mit dem bekannten Verfahren hergestellten Struktur relativ "weich" dadurch, daß nur abgerundete Erhebungen 2 nach außen ragen.

## Patentansprüche

1. Verfahren zur Herstellung offenzelliger metallischer Strukturen unter Anwendung eines verlorenen Positivmodells aus einem offenporigen Kunststoffträger mit einer Durchschnittsweite der Poren von 1 bis 3 mm, bei dem nach Herstellung eines Negativmodells dessen Kernräume durch Gießen mit Metall gefüllt werden und schließlich das Negativmodell entfernt wird, gekennzeichnet durch die folgenden Verfahrensschritte:
a) Nach erfolgter Reinigung des Kunststoffträgers wird dieser durchgängig mit einem seine Oberflächenstrukturen anlösenden haftungsvermittelnden Harzfilm benetzt, und
b) es wird mindestens eine Schicht eines selbstvernetzenden Zweikomponenten-Silikons durchgängig in bzw. auf den Kunststoffträger ein- bzw. aufgetragen zur Verstärkung der Wandungen und/oder vernetzten Stege des porigen Kunststoffträgers.

2. Verfahren nach Anspruch 1, gekennzeichnet durch den folgenden zusätzlichen Verfahrensschritt:
c) Nach Vernetzung des Zweikomponenten-Silikons wird die Oberflächenstruktur zusätzlich durch ein Zweikomponenten-Polyurethanharz versteift.

3. Verfahren nach Anspruch 1 oder 2, dadurch gekennzeichnet, daß der haftungsvermittelnde Harzfilm aus einem ungesättigten Silikonharz besteht.

4. Verfahren nach einem der Ansprüche 1 bis 3, dadurch gekennzeichnet, daß das Zweikomponenten-Silikon ein raum temperaturvernetzender, ungesättigter Zweikomponenten-Silikonkautschuk ist.

5. Verfahren nach einem der Ansprüche 1 bis 4, dadurch gekennzeichnet, daß mehr als eine Schicht des Zweikomponenten-Silikons in bzw. auf den Kunststoffträger ein- bzw. aufgetragen wird, wobei die einzelnen Schichten verschiedenfarbig sind, um Schichtdicken erkennbar zu machen .

## Claims

1. Process for producing open-celled metal structures using a lost positive model comprising an open-pore plastic substrate having pores with an average width of 1 to 3 mm, in which process after producing a negative model, the voids of which are filled with metal by casting,and then the negative model is removed, characterised by the following process steps:
a) after the plastic substrate has been cleaned, it is thoroughly moistened with an adhesion-promoting resin film partially dissolving its surface structures, and
b) at least one layer of a self-crosslinking two-component silicone is introduced into or applied onto the plastic substrate thoroughly to strengthen the walls and/or interlinked webs of the porous plastic substrate.

2. Process according to claim 1, characterised by the following additional process step:
c) after crosslinking the two-component silicone, the surface structure is additionally stiffened by means of a two-component polyurethane resin.

3. Process according to claim 1 or 2, characterised in that the adhesion-promoting resin film consists of an unsaturated silicone resin.

4. Process according to one of claims 1 to 3, characterised in that the two-component silicone is an unsaturated two-component silicone rubber which crosslinks at room temperature.

5. Process according to one of claims 1 to 4, characterised in that more than one layer of the two-component silicone is introduced into or applied onto the plastic substrate, the individual layers having different colours to make the layer thicknesses distinguishable.

## Revendications

1. Procédé de fabrication de structures métalliques à alvéoles ouverts en utilisant un modèle positif perdu formé d'un support en plastique à pores ouverts avec un diamètre de pores de 1 à 3 mm, dans lequel, après la fabrication d'un modèle négatif, les espaces du noyau de celui-ci sont remplis de métal par coulée et le modèle négatif est ensuite retiré, caractérisé en ce qu'il comporte les étapes de procédé suivantes :
a) une fois le support de plastique nettoyé, celui-ci est entièrement recouvert d'un film de résine provoquant un mordançage de ses structures de surface et conférant une adhérence, et
b) au moins une couche d'un silicone à deux composants auto-réticulant est appliquée entièrement dans et sur le support en plastique pour renforcer les parois et/ou les ponts réticulés du support en plastique poreux.

2. Procédé selon la revendication 1, caractérisé en ce qu'il comporte l'étape de procédé supplémentaire suivante:
c) Une fois le silicone à deux composants réticulé, la structure de surface est en outre raidie par une résine polyuréthane à deux composants.

3. Procédé selon la revendication 1 ou 2, caractérisé en ce que le film de résine conférant une adhérence se compose d'une résine silicone insaturée.

4. Procédé selon l'une quelconque des revendications 1 à 3, caractérisé en ce que le silicone à deux composants est un caoutchouc siliconé à deux composants insaturé réticulant à température ambiante.

5. Procédé selon l'une quelconque des revendications 1 à 4, caractérisé en ce que plusieurs couches du silicone à deux composants sont appliquées dans et sur le support en plastique, les différentes couches étant de couleur différente, pour rendre les épaisseurs de couches reconnaissables.
